(19) Europäisches Patentamt / European Patent Office / Office européen des brevets

(11) **EP 3 614 145 A1**

(12) **EUROPEAN PATENT APPLICATION**
published in accordance with Art. 153(4) EPC

(43) Date of publication:
**26.02.2020 Bulletin 2020/09**

(21) Application number: **18786990.4**

(22) Date of filing: **28.02.2018**

(51) Int Cl.:
*G01N 33/50* (2006.01)    *C12Q 1/02* (2006.01)
*G01N 33/15* (2006.01)

(86) International application number:
**PCT/JP2018/007559**

(87) International publication number:
**WO 2018/193722 (25.10.2018 Gazette 2018/43)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **20.04.2017 JP 2017083741**

(71) Applicant: **Mandom Corporation**
**Osaka-shi, Osaka 540-8530 (JP)**

(72) Inventors:
• **ISHIZUKA Shuta**
**Osaka-shi**
**Osaka 540-8530 (JP)**
• **KURATA Ryuichiro**
**Osaka-shi**
**Osaka 540-8530 (JP)**

(74) Representative: **TBK**
**Bavariaring 4-6**
**80336 München (DE)**

(54) **METHOD FOR OBSERVING SEBACEOUS GLAND**

(57) A method for observing a sebaceous gland, the method being useful for such applications as developing a cosmetic material or other external preparation, and including (I) a step for culturing, in a suspended state in a medium, a sebaceous gland structure in which the der-mis and all or a portion of the subcutaneous tissue is removed from a skin tissue, and (II) a step for observing the cultured sebaceous gland structure obtained in step (I).

EP 3 614 145 A1

**Description**

TECHNICAL FIELD

The present invention relates to a method for observing a sebaceous gland. More specifically, the present invention relates to a method for observing a sebaceous gland and a method for evaluating a test substance, which are useful for development of external preparations such as a cosmetic.

BACKGROUND ART

Sebum is secreted from a sebaceous gland onto the surface of skin. The sebum shows a skin-protecting action on a stimulus from outside of the body. However, when the amount of sebum present in the skin is too small, the sebum cannot sufficiently protect the skin in some cases. On the other hand, when the amount of sebum present in the skin is excessive, acne, seborrheic dermatitis, sebaceous hyperplasia and the like can be caused in some cases. Accordingly, external preparations which are used for appropriately regulating sebum production such as a cosmetic have been desired.

On the other hand, influence of the substance on sebum production has been analyzed by a culture test using a sebocyte (for example, see Patent Literature 1). However, since the culture test using a sebocyte cannot sufficiently reproduce sebum production from a sebaceous gland in a living body, the culture test has a disadvantage that it is difficult to accurately observe dynamics of a sebaceous gland in a living body.

PRIOR ART LITERATURES

PATENT LITERATURES

Patent Literature 1: Japanese Unexamined Patent Publication No. 2013-32331

SUMMARY OF THE INVENTION

PROBLEMS TO BE SOLVED BY THE INVENTION

The present invention has been accomplished in view of the above-mentioned prior art. An object of the present invention is to provide a method for observing a sebaceous gland, which can accurately observe dynamics of a sebaceous gland in a living body, and a method for evaluating a test substance, which can accurately evaluate whether or not the test substance is a sebum production-regulating substance.

MEANS FOR SOLUVING THE PROBLEMS

In summary, the present invention relates to:

(1) a method for observing a sebaceous gland, including the steps of:

(I) culturing, in a suspended state in a medium, a sebaceous-gland-structure derived from a skin tissue, in which all or a portion of each of dermis and a subcutaneous tissue has been removed from the skin tissue, and
(II) observing the resulting cultured sebaceous-gland-structure in step (I);

(2) the method for observing a sebaceous gland according to the item (1), further including a step of removing all or the portion of each of the dermis and the subcutaneous tissue from an isolated skin tissue, to obtain the sebaceous-gland-structure, before carrying out the step (I); and

(3) a method for evaluating a sebum production-regulating action of a test substance, including the steps of:

(A) culturing, in a suspended state in a medium in the absence of the test substance, a sebaceous-gland-structure derived from a skin tissue, in which all or a portion of each of dermis and a subcutaneous tissue has been removed from the skin tissue,
(B) culturing, in a suspended state in a medium in the presence of the test substance, a sebaceous-gland-structure derived from a skin tissue, in which all or a portion of each of dermis and a subcutaneous tissue has been removed from the skin tissue, and
(C) observing the resulting cultured sebaceous-gland-structure (A) in the step (A) and the resulting cultured sebaceous-gland-structure (B) in the step (B), and evaluating the sebum production-regulating action of the test substance on the basis of difference in dynamics of the sebaceous gland in the sebaceous-gland-structure (A) and dynamics of the sebaceous gland in the sebaceous-gland-structure (B).

EFFECTS OF THE INVENTION

According to the method for observing a sebaceous gland of the present invention, an excellent effect that dynamics of a sebaceous gland in a living body can be accurately observed is exhibited. In addition, according to the method for evaluating a test substance of the present invention, an excellent effect that whether or not the test substance is a sebum production-regulating substance can be accurately evaluated is exhibited.

BRIEF DESCRIPTION OF THE DRAWINGS

Fig. 1 is a schematic explanation view showing the main part of a sebaceous-gland-structure, which contains a sebaceous gland.
Fig. 2 is a photograph substituted for a drawing, showing results of observation in Test Example 1(1) of appearance of the sebaceous-gland-structure A contained in an observation sample obtained in Preparation Example 7.
Fig. 3 is a photograph substituted for a drawing, showing results of observation in Test Example 1(1) of appearance of the sebaceous-gland-structure A contained in an observation sample obtained in Preparation Example 7.
Fig. 4 is a photograph substituted for a drawing, showing results of observation in Test Example 1(2) of the inside of the sebaceous-gland-structure A contained in an observation sample obtained in Preparation Example 7.
Fig. 5 is a photograph substituted for a drawing, showing results of observation in Test Example 1(2) of lipid droplets of sebum in the inside of the sebaceous-gland-structure A contained in an observation sample obtained in Preparation Example 7.
Fig. 6 is a photograph substituted for a drawing, showing results of classification and observation in Example 1(5) of the state of lipid droplets in cells contained in the outermost layer of the sebaceous gland of the sebaceous-gland-structure A contained in an observation sample of each of Experiment numbers 1 to 6.
Fig. 7(A) is a photograph substituted for a drawing, showing results of observation in Example 2(3) of the inside of the sebaceous-gland-structure A contained in an observation sample of Experiment number 7, and (B) is a photograph substituted for a drawing, showing the enlarged part of the observed region surrounded with a frame in (A).
Fig. 8 (A) is a photograph substituted for a drawing, showing results of observation of the inside of the sebaceous-gland-structure A contained in an observation sample of Experiment number 8 in Example 2(3), and (B) is a photograph substituted for a drawing, showing the enlarged part of the observed region surrounded with a frame in (A).
Fig. 9 (A) is a photograph substituted for a drawing, showing results of observation of the inside of the sebaceous-gland-structure A contained in an observation sample of Experiment number 9 in Example 2(3), and (B) is a photograph substituted for a drawing, showing the enlarged part of the observed region surrounded with a frame in (A).
Fig. 10 is a graph showing results of examination of the relationship between the kind of sample and the degree of progress of sebum production in Example 2(3).
Fig. 11 is a graph showing results of examination of the relationship between the kind of sample and the degree of sebum production in Example 4(4).

MODE FOR CARRING OUT THE INVENTION

In one aspect, the present invention relates to a method for observing a sebaceous gland, including the steps of:

(I) culturing, in a suspended state in a medium, a sebaceous-gland-structure derived from a skin tissue, in which all or a portion of each of dermis and a subcutaneous tissue has been removed from the skin tissue, and
(II) observing the resulting cultured sebaceous-gland-structure in step (I).

According to the method for observing a sebaceous gland of the present invention, since a process for culturing the sebaceous-gland-structure in a suspended state in a medium is employed, dynamics of a sebaceous gland in a living body, which is represented by sebum production from a sebaceous gland of a living body can be accurately reproduced in a state easy to observe in the sebaceous-gland-structure even if the sebaceous-gland-structure lacks all or a portion of each of dermis and a subcutaneous tissue. Accordingly, according to the method for observing a sebaceous gland of the present invention, dynamics of a sebaceous gland can be observed in an environment closer to in vivo environment.

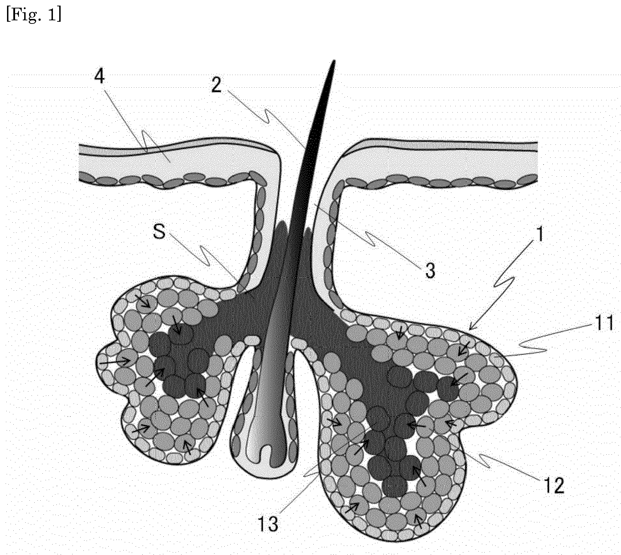

The sebaceous-gland-structure is a structure derived from a skin tissue, in which all or a portion of each of dermis and a subcutaneous tissue has been removed from the skin tissue. More specifically, as shown in Fig. 1, the sebaceous-gland-structure contains a sebaceous gland 1, a hair 2, a hair follicle 3 enclosing the hair 2, and epidermis 4. The sebaceous gland 1 contains an undifferentiated sebaceous gland basal cell 11 which is localized to the outermost layer of the sebaceous gland 1, a lipid droplet-generating cell 12 (hereinafter also referred to as "mature sebocyte") which is localized inside the localized position of the undifferentiated sebaceous gland basal cell 11, and cell debris 13 which is caused by cell death and disintegration of the mature sebocyte and which is localized to the central part of the sebaceous gland 1. Sebum S generated in the sebaceous gland 1 is secreted to the outside of the skin through the hair follicle 3. A part constituted by the sebaceous gland 1, the hair 2 and the hair follicle 3 in the sebaceous-gland-structure has the same structure as that of a part constituted by a sebaceous gland, a hair, a hair follicle and epidermis in a skin tissue in a living body, except that all or a portion of each of a dermis and a subcutaneous tissue is removed from the skin tissue.

In the present specification, "all or a portion of each of the dermis and the subcutaneous tissue is removed from the skin tissue" means that the dermis and the subcutaneous tissue are removed from the skin tissue and the sebaceous gland is exposed to outside so that the sebaceous gland can be observed.

In the step (I), the sebaceous-gland-structure is cultured in a suspended state in a medium. The sebaceous-gland-structure can be produced by, for example, removing all or a portion of each of dermis and a subcutaneous tissue from an isolated skin tissue. Accordingly, the method for observing a sebaceous gland of the present invention can further include a step of removing all or the portion of each of the dermis and the subcutaneous tissue from the isolated skin tissue, before carrying out the step (I).

The method for producing the sebaceous-gland-structure includes, for example, a method including the steps of:

(a1) excising all or a portion of a subcutaneous tissue from an isolated skin tissue, and
(a2) removing a fiber such as a collagenous fiber from the tissue obtained in the step (a1) to expose a sebaceous gland to outside, thereby giving the sebaceous-gland-structure (hereinafter also referred to as "production method A");
(b1) excising all or a portion of a subcutaneous tissue from an isolated skin tissue,
(b2) contacting the tissue obtained in the step (b1) with an enzyme for dissociating an epidermal appendage from the dermis such as dispase, collagenase and pronase, and
(b3) separating the dermis from the tissue obtained in the step (b2) (hereinafter also referred to as "production method B"), and the like, and the present invention is not limited only to those exemplified ones. Among these production methods of a sebaceous-gland-structure, the production method A and the production method B are preferable, and the production method A is more preferable, since a sebaceous-gland-structure can be easily produced and dynamics of a sebaceous gland in a living body can be accurately observed.

The isolated skin tissue includes, for example, a living skin tissue obtained from excessive skin generated during a surgical operation, and the like, and the present invention is not limited only to those exemplified ones. Additionally, in the present specification, the "living skin tissue" refers to a skin tissue exhibiting the same biological activity as an inherent biological activity and the same movement as an inherent movement in a living body.

The source of the skin tissue includes, for example, a human and the like, and the present invention is not limited only to those exemplified ones. When the method for observing a sebaceous gland of the present invention is used for observation of a sebaceous gland at the time of sebum production in a human, and the like, it is preferable that the source of the skin tissue is a human. Conventionally, it has been difficult to accurately observe dynamics of a human sebaceous gland. Accordingly, the method for observing a sebaceous gland of the present invention is suitable for observation of a human sebaceous gland.

Dynamics of a sebaceous gland includes, for example, a change in the shape of a sebaceous gland-constituting cell when the shapes of the cell are compared between at least two time points upon sebum production, a change in the differentiated state of a sebaceous gland-constituting cell when the differentiated states of the cell are compared between at least two time points upon sebum production, a change in the shape of a differentiated cell when the shapes of the differentiated cell are compared between at least two time points upon sebum production, a change in a position of the localized region of a differentiated cell when the positions of the localized regions of the differentiated cells are compared between at least two time points upon sebum production, and the like, and the present invention is not limited only to those exemplified ones.

The sebaceous-gland-structure is cultured in a state of suspending the sebaceous-gland-structure in a medium. The "state of suspending the sebaceous-gland-structure in a medium" is not particularly limited, as long as the sebaceous-gland-structure does not contact with the wall surface of a culture container used for culture of the sebaceous-gland-structure. Accordingly, the "state of suspending the sebaceous-gland-structure in a medium" can be a state where the whole sebaceous-gland-structure is immersed in the medium or a state where a part of the sebaceous-gland-structure is exposed from the medium and the remaining part is immersed in the medium, as long as the sebaceous-gland-structure does not contact with the wall surface of a culture container used for culture of the sebaceous-gland-structure.

The medium is not particularly limited, as long as the medium contains a differentiation-promoting component suitable for differentiation of an undifferentiated sebaceous gland basal cell contained in the sebaceous-gland-structure into a mature sebocyte and a growth component for proliferating a cell. The medium can be a medium obtained by supplementing a conventional basal medium supplemented with the differentiation-promoting component and the growth component, or a commercially available medium. The differentiation-promoting component includes, for example, fetal bovine serum, a fatty acid, a peptide, a hormone and the like, and the present invention is not limited only to those exemplified ones. These differentiation-promoting components can be used alone, or in combination of two or more kinds of the components. The content of the differentiation-promoting component in the medium cannot be absolutely determined because the content varies depending on the kind of the medium, the kind of the differentiation-promoting component and the like. It is therefore preferred to appropriately adjust the content in accordance with the kind of the medium, the kind of the differentiation-promoting component, and the like. The growth component includes, for example, an amino acid, a vitamin, an inorganic salt, a saccharide, a cell growth factor and the like, and the present invention is not limited only to those exemplified ones. The content of the growth component in the medium cannot be absolutely determined because the content varies depending on the kind of the medium, the kind of the growth component, and the like. It is therefore preferred to appropriately adjust the content in accordance with the kind of the medium, the kind of the growth component, and the like. These growth components can be used alone, or in combination of two or more kinds of the components. The basal medium includes, for example, a mixed medium of Dulbecco's modified Eagle's medium and F-12 medium, and the like, and the present invention is not limited only to those exemplified ones.

The culture conditions upon culturing the sebaceous-gland-structure cannot be absolutely determined because the culture conditions vary depending on the kind of the source of the skin tissue to be used for producing the sebaceous-gland-structure, the kind of dynamics of the sebaceous gland to be observed, and the like. It is therefore preferred to appropriately adjust the culture conditions in accordance with the kind of the source of the skin tissue to be used for producing the sebaceous-gland-structure, the kind of dynamics of the sebaceous gland to be observed, and the like. The culture conditions include, for example, culture temperature, culture time, pH of the medium, carbon dioxide concentration in the culture atmosphere, and the like.

In general, culture temperature is generally preferably 35 to 38°C, and more preferably 36.5 to 37.5°C, from the viewpoint of accurate reproduction of the state of a sebaceous gland in a living body, when the source of the skin tissue is a human. In addition, in general, culture time is generally preferably 6 to 168 hours, and more preferably 24 to 48 hours from the viewpoint of maintenance of physiological functions in good conditions in the sebaceous-gland-structure, when the source of the skin tissue is a human. Furthermore, in general, pH of the medium is generally preferably 6.8 to 7.6, and more preferably 7.0 to 7.4, from the viewpoint of accurate reproduction of the state of a sebaceous gland in a living body, when the source of the skin tissue is a human. In general, carbon dioxide concentration in the culture atmosphere is preferably 4 to 10% by volume, and more preferably 5 to 7% by volume, from the viewpoint of accurate reproduction of the state of a sebaceous gland in a living body.

Next, in the step (II), the cultured sebaceous-gland-structure is observed. The cultured sebaceous-gland-structure can be observed by, for example, using as an observation sample, the sebaceous-gland-structure prepared by staining the cultured sebaceous-gland-structure with a staining reagent, and the like.

The sebaceous-gland-structure can be stained by, for example, contacting the sebaceous-gland-structure with the staining reagent, and the like. The cultured sebaceous-gland-structure used for the staining can be a fixed sample prepared by fixing the sebaceous-gland-structure with a fixing solution. The fixed sample can be any sample, as long as the cell membrane of each cell contained in the sebaceous-gland-structure has permeability sufficient for permeating the staining reagent through the cell membrane. The sebaceous-gland-structure is fixed by, for example, adding a fixation solution to a culture container containing a sebaceous-gland-structure, to contact the sebaceous-gland-structure with the fixation solution, and the like.

The fixation solution includes, for example, acetone, methanol, a mixed solution of acetone and methanol, an aqueous formaldehyde solution, a formaldehyde phosphate buffer solution, an aqueous paraformaldehyde solution, a paraformaldehyde phosphate buffer solution and the like, and the present invention is not limited only to those exemplified ones. Prior to staining, the fixed sample can be washed with an appropriate washing liquid as occasion demands, or cannot be washed. The washing liquid includes, for example, phosphate-buffered saline, tris-buffered saline and the like, and the present invention is not limited only to those exemplified ones.

The staining reagent includes, for example, a reagent containing a complex of a binding substance which binds to a marker and a detectable substance, a reagent which contains the binding substance but does not contain the detectable substance, and the like, and the present invention is not limited only to those exemplified ones. In the present specification, the "marker" refers to a substance which serves as an index of presence of a tissue, a cell and the like contained in the sebaceous-gland-structure, an index of the degree of differentiation of a cell, and the like.

The binding substance cannot be absolutely determined because the binding substance varies depending on a use of the method for observing a sebaceous gland of the present invention and the like. It is therefore preferable to appropriately determine the binding substance in accordance with the use of the method for observing a sebaceous gland of the present invention, and the like. The binding substance includes, for example, an antibody which binds to the marker (hereinafter simply referred to as "antibody") or a fragment thereof (hereinafter simply referred to as "antibody fragment"), a compound which binds to the marker, and the like, and the present invention is not limited only to those exemplified ones. The antibody can be a polyclonal antibody or a monoclonal antibody. Among the antibodies, a monoclonal antibody is preferable, since specificity to the marker is high. The antibody fragment includes, for example, a Fab fragment, a F(ab')$_2$ fragment, a single-chain antibody and the like, and the present invention is not limited only to those exemplified ones. The polyclonal antibody, the monoclonal antibody and the antibody fragment can be produced by using the marker as an antigen in accordance with a conventional technique. In addition, as the polyclonal antibody, the monoclonal antibody and the antibody fragment, a commercially easily available polyclonal antibody, a commercially easily available monoclonal antibody and a commercially easily available antibody fragment can be used. The binding substance can be a substance which generates a detectable signal or a substance which does not generate a detectable signal.

The detectable substance includes, for example, a fluorescent substance, an enzyme and the like, and the present invention is not limited only to those exemplified ones. The fluorescent substance includes, for example, fluorescein isothiocyanate, a fluorescent substance of Alexa Fluor series (for example, manufactured by Invitrogen, trade name: Alexa Fluor 647 and the like) and the like, and the present invention is not limited only to those exemplified ones. In addition, the enzyme includes, for example, peroxidase, alkaline phosphatase and the like, and the present invention is not limited only to those exemplified ones. Among these detectable substances, a fluorescent substance is preferable, and a fluorescent substance of Alexa Fluor series (for example, manufactured by Invitrogen, trade name: Alexa Fluor 647 and the like) is more preferable, since detection operation can be easily operated and an object to be detected can be detected with high accuracy.

When the binding substance is an antibody or an antibody fragment thereof, the staining reagent can further contain a labeled binding substance which binds to the antibody or the antibody fragment thereof, as occasion demands. The labeled binding substance includes, for example, a complex of a second binding substance which binds to the binding substance and a labeling substance, and the like, and the present invention is not limited only to those exemplified ones. The second binding substance includes, for example, an antibody against an immunoglobulin of an animal immunized upon producing the antibody, an antibody against a fragment of the immunoglobulin, and the like, and the present invention is not limited only to those exemplified ones. The labelling substance includes, for example, the detectable substances and the like, and the present invention is not limited only to those exemplified ones.

The kind of the staining reagent cannot be absolutely determined because the kind varies depending on a use of the method for observing a sebaceous gland of the present invention and the like. It is therefore preferred to appropriately determine the kind in accordance with the use of the method for observing a sebaceous gland of the present invention and the like. The staining reagent includes, for example, a staining reagent for the undifferentiated sebaceous gland basal cell (hereinafter also referred to as "undifferentiated cell-staining reagent"), a staining reagent for sebum (hereinafter also referred to as "sebum-staining reagent"), a staining reagent for a cell nucleus (hereinafter also referred to as "nucleus-staining reagent"), a staining reagent for a differentiated cell, and the like, and the present invention is not limited only to those exemplified ones.

The undifferentiated cell-staining reagent contains a substance (hereinafter also referred to as "undifferentiated cell-binding substance") which binds to a marker specific for an undifferentiated sebaceous gland basal cell (hereinafter also referred to as "undifferentiation marker"). The undifferentiated cell-staining reagent can be a reagent containing a complex of an undifferentiated cell-binding substance and a detectable substance. When the undifferentiated cell-binding substance itself generates a detectable signal, the undifferentiated cell-staining reagent can be a reagent which contains the undifferentiated cell-binding substance but does not contain the detectable substance. The undifferentiated cell marker includes, for example, keratin-5, keratin-7, keratin-14, B lymphocyte-induced maturation protein-1 (Blimp1), leucine-rich repeat and immunoglobulin-like domain protein-1 (Lrig1) and the like, and the present invention is not limited only to those exemplified ones.

The nucleus-staining reagent contains a binding substance which binds to a nucleus-constituting substance (hereinafter also referred to as "nucleus-binding substance"). The nucleus-staining reagent can be a reagent containing a complex of the nucleus-binding substance and a detectable substance. When the nucleus-binding substance itself is a substance which generates a detectable signal, the nucleus-staining reagent can be a reagent which contains the nucleus-binding substance but does not contain the detectable substance. The nucleus-constituting substance includes, for example, a nucleic acid such as DNA, and the like, and the present invention is not limited only to those exemplified ones. The nucleus-binding substance can be any substance which permeates through a cell membrane. The nucleus-binding substance includes, for example, Hoechst 33342 and the like, and the present invention is not limited only to those exemplified ones. Since Hoechst 33342 generates detectable fluorescence, the nucleus-staining reagent can be a reagent which does not contain the detectable substance.

The sebum-staining reagent includes, for example, a lipophilic dye which dissolves in sebum, and the like, and the present invention is not limited only to those exemplified ones. The lipophilic dye includes, for example, Nile red, Nile blue, Oil red O, Sudan III, Sudan IV, Sudan black B and the like, and the present invention is not limited only to those exemplified ones. Since the lipophilic dye dissolves in sebum to stain the sebum, a sebum-staining reagent which contains the lipophilic dye can be a reagent which does not contain the detectable substance.

The differentiated cell-staining reagent contains, for example, a substance (hereinafter also referred to as "differentiated cell-binding substance") which binds to a marker specific for a sebocyte differentiated from an undifferentiated sebum gland basal cell (hereinafter also referred to as "differentiation marker"). The differentiated cell-staining reagent can be a reagent containing a complex of a differentiated cell-binding substance and a detectable substance. When the differentiated cell-binding substance itself generates a detectable signal, the differentiated cell-staining reagent can be a reagent which contains the differentiated cell-binding reagent but does not contain the detectable substance. The differentiated cell marker includes, for example, stearyl-CoA desaturase-1 (Scd-1), peroxisome proliferator-activated receptor-$\gamma$ (PPAR$\gamma$) and the like, and the present invention is not limited only to those exemplified ones.

The content of the binding substance in the staining reagent cannot be absolutely determined because the content varies depending on the kind of the binding substance, a use of the method for observing a sebaceous gland of the present invention and the like. It is therefore preferred to appropriately adjust the content in accordance with the kind of the binding substance, the use of the method for observing a sebaceous gland of the present invention, and the like.

It is preferable that the detectable substance contained in the undifferentiated cell-staining reagent, the detectable substance contained in the sebum-staining reagent and the detectable substance contained in the nucleus-staining reagent can be distinguished from each other.

The mixing ratio and contact time of the sebaceous-gland-structure and the staining reagent cannot be absolutely determined because the mixing ratio and contact time vary depending on the kind of the staining reagent and the like. It is therefore preferred to appropriately adjust the mixing ratio and contact time in accordance with the kind of the staining reagent and the like.

After contacting the sebaceous-gland-structure with the staining reagent, it is preferable to wash a stained sebaceous-gland-structure with an appropriate washing liquid, from the viewpoint of more accurate observation of dynamics of a sebaceous gland. The washing liquid includes, for example, phosphate-buffered saline, phosphate buffer and the like, and the present invention is not limited only to those exemplified ones.

When the staining reagent contains an antibody or an antibody fragment thereof, it is preferred that the stained sebaceous-gland-structure is subjected to a blocking treatment using a blocking agent, from the viewpoint of more accurate observation of dynamics of a sebaceous gland. The blocking agent includes, for example, phosphate-buffered saline containing albumin, and the like, and the present invention is not limited only to those exemplified ones.

Dynamics of a sebaceous gland is observed by using the stained sebaceous-gland-structure. The sebaceous gland can be observed by using, for example, an optical microscope such as a fluorescence microscope or a confocal laser microscope. More specifically, the sebaceous gland can be observed by, for example, directly subjecting the stained sebaceous-gland-structure to observation, detecting a signal derived from the staining reagent in the stained sebaceous-gland-structure, and the like.

As explained above, according to the method for observing a sebaceous gland of the present invention, sebum production from a sebaceous gland can be accurately observed. It is therefore expected that the method is used for screening of a sebum production-regulating substance which regulates sebum production, evaluation of the effectiveness of a sebum production-regulating substance, and the like.

2. Method for Evaluating Test Substance

In another aspect, the present invention relates to a method for evaluating a sebum production-regulating action of a test substance, including the steps of:

(A) culturing, in a suspended state in a medium in the absence of the test substance, a sebaceous-gland-structure derived from a skin tissue, in which all or a portion of each of dermis and a subcutaneous tissue has been removed from the skin tissue,
(B) culturing, in a suspended state in a medium in the presence of the test substance, a sebaceous-gland-structure derived from a skin tissue, in which all or a portion of each of dermis and a subcutaneous tissue has been removed from the skin tissue, and
(C) observing the resulting cultured sebaceous-gland-structure (A) in the step (A) and the resulting cultured sebaceous-gland-structure (B) in the step (B), and evaluating the sebum production-regulating action of the test substance on the basis of difference in dynamics of the sebaceous gland in the sebaceous-gland-structure (A) and dynamics of the sebaceous gland in the sebaceous-gland-structure (B).

In the method for evaluating a test substance of the present invention, since a process for carrying out the steps (A) and (B), and observing the resulting cultured sebaceous-gland-structure (A) obtained in the step (A) and the resulting cultured sebaceous-gland-structure (B) obtained in the step (B) is employed, sebum production-regulating action of the test substance can be accurately evaluated. Thus, according to the method for evaluating a test substance of the present invention, whether or not the test substance is a sebum production-regulating substance can be accurately evaluated. The sebum production-regulating action includes a sebum production-promoting action which increases sebum production and a sebum production-inhibiting action which decreases sebum production. The order of process for carrying out the steps (A) and (B) can be an order of process for carrying out the step (A) and thereafter carrying out the step (B), or can be an order of process for carrying out the step (B) and thereafter carrying out the step (A). In addition, the steps (A) and (B) can be carried out at the same time.

First, in the step (A), a sebaceous-gland-structure derived from a skin tissue, in which all or a portion of each of dermis and a subcutaneous tissue has been removed from the skin tissue is cultured in a suspended state in a medium in the absence of the test substance. The sebaceous-gland-structure and the medium used in the step (A) are the same as those used in the method for observing a sebaceous gland described above. The sebaceous-gland-structure in the step (A) can be cultured in accordance with the same technique as that used for the culture of the sebaceous-gland-structure in the method for observing a sebaceous gland described above. The culture conditions of the sebaceous-gland-structure cannot be absolutely determined because culture conditions vary depending on the evaluation content, the kind of the test substance to be evaluated, the kind of the test substance and the source of the skin tissue used for producing the sebaceous-gland-structure, and the like. It is therefore preferred to appropriately adjust the culture conditions in accordance with the evaluation content, the kind of the test substance to be evaluated, the kind of the source of the skin tissue used for producing the sebaceous-gland-structure, and the like.

In the step (B), a sebaceous-gland-structure derived from a skin tissue, in which all or a portion of each of dermis and a subcutaneous tissue has been removed from the skin tissue is cultured in a suspended state in a medium in the presence of the test substance. The sebaceous-gland-structure used in the step (B) is obtained from a skin tissue of the same source as that of the sebaceous-gland-structure used in the step (A) and is the same kind of sebaceous-gland-structure as that used in the step (A). However, the sebaceous-gland-structure used in the step (B) is different from the sebaceous-gland-structure used in the step (A). The medium used in the step (B) is the same kind of medium as that used in the step (A). In the step (B), the sebaceous-gland-structure can be cultured in the presence of a test substance by, for example, the same technique as that used for the culture of the sebaceous-gland-structure in the method for observing a sebaceous gland described above, except that a test substance-containing medium obtained by adding a test substance to the same kind of medium as that used in the step (A). The concentration of the test substance in the test substance-containing medium cannot be absolutely determined because the concentration varies depending on the evaluation contents, the kind of the test substance to be evaluated, the kind of the source of the skin tissue used for producing the sebaceous-gland-structure, and the like. It is therefore preferred to appropriately adjust the content in accordance with the evaluation contents, the kind of the test substance to be evaluated, the kind of the source of the skin tissue used for producing the sebaceous-gland-structure, and the like. Culture conditions of the sebaceous-gland-structure in the step (B) are the same as those of the sebaceous-gland-structure in the step (A), except that a test substance is not used.

In the step (C), the resulting cultured sebaceous-gland-structure (A) in the step (A) and the resulting cultured sebaceous-gland-structure (B) in the step (B) are observed to evaluate the sebum production-regulating action of the test substance on the basis of difference between dynamics of a sebaceous gland in the sebaceous-gland-structure (A) and dynamics of a sebaceous gland in the sebaceous-gland-structure (B). The observation technique of a sebaceous-gland-structure in the step (C) is the same as that of a sebaceous-gland-structure in the method for observing a sebaceous gland described above.

The difference between dynamics of a sebaceous gland in the sebaceous-gland-structure (A) and dynamics of a sebaceous gland in the sebaceous-gland-structure (B) includes a difference in the number of cells containing sebum in an outermost layer of a sebaceous gland depending on the presence or absence of the test substance, a difference in differentiation speed of an undifferentiated sebaceous gland basal cell into a mature sebocyte in a sebaceous gland depending on the presence or absence of the test substance, a difference in the number of disintegrated mature sebocytes present near a central part of a sebaceous gland depending on the presence or absence of the test substance, a difference in an amount of sebum present near a central part of a sebaceous gland depending on the presence or absence of the test substance, a difference in a position where a differentiation marker is expressed depending on the presence or absence of the test substance, a difference in an amount of expression of a differentiation marker depending on the presence or absence of the test substance, and the like, but the present invention is not limited only to those exemplified ones.

An index showing that the test substance has a sebum production-promoting action includes, for example, indices (1a) to (3a) described below, and the like. These indices can be used alone, or in combination of two or more kinds of indices.

(1a) the number of cells containing sebum in the outermost layer of a sebaceous gland of the sebaceous-gland-structure (B) is larger than the number of cells containing sebum in the outermost layer of a sebaceous gland of the sebaceous-gland-structure (A);

(2a) the differentiation speed of an undifferentiated sebaceous gland basal cell in a sebaceous gland in the sebaceous-gland-structure (B) into a mature sebocyte is higher than the differentiation speed of an undifferentiated sebaceous gland basal cell in a sebaceous gland in the sebaceous-gland-structure (A) into a mature sebocyte; and

(3a) the number of disintegrated mature sebocytes present near the central part of a sebaceous gland of the sebaceous-gland-structure (B) is larger than the number of disintegrated mature sebocytes present near the central part of a sebaceous gland of the sebaceous-gland-structure (A).

In addition, an index showing that the test substance has a sebum production-inhibiting action includes, for example, indices (1b) to (3b) described below, and the like. These indices can be used alone, or in combination of two or more kinds of indices.

(1b) the number of cells containing sebum in the outermost layer of a sebaceous gland of the sebaceous-gland-structure (B) is smaller than the number of cells containing sebum in the outermost layer of a sebaceous gland of the sebaceous-gland-structure (A);

(2b) the differentiation speed of an undifferentiated sebaceous gland basal cell in a sebaceous gland in the sebaceous-gland-structure (B) into a mature sebocyte is lower than the differentiation speed of an undifferentiated sebaceous gland basal cell in a sebaceous gland in the sebaceous-gland-structure (A) into a mature sebocyte; and

(3b) the number of disintegrated mature sebocytes present near the central part of a sebaceous gland of the sebaceous-gland-structure (B) is smaller than the number of disintegrated mature sebocytes present near the central part of a sebaceous gland of the sebaceous-gland-structure (A).

As explained above, according to the method for evaluating a test substance of the present invention, a sebum production-regulating action of the test substance can be accurately evaluated. It is therefore expected that the method is used for screening of a sebum production-regulating substance, evaluation of effectiveness of a sebum production-regulating substance, and the like. It is expected that the sebum production-regulating substance is used for, for example, a sebum production-inhibiting agent, a sebum production-promoting agent and the like which are formulated in a cosmetic used for the skin of a face, an armpit, a scalp or the like.

EXAMPLES

Hereinafter, the present invention will be described in more detail below, but the present invention is not limited only to the working examples. Hereinafter, the meanings of abbreviations are as follows.

<Explanation of Abbreviations>

BSA: bovine serum albumin
DMEM: Dulbecco's modified Eagle's medium
FBS: fetal bovine serum
PBS: phosphate buffered saline
PBSTT: a PBS solution containing polyoxyethylene sorbitan monolaurate (Tween 20) and polyoxyethylene (10) octylphenyl ether (Triton X-100) (composition: 0.1% by volume of Tween 20, 0.5% by volume of Triton X-100 and the balance being PBS)
PFA: paraformaldehyde

Preparation Example 1

FBS and an extracellular matrix (manufactured by Corning Incorporated, trade name: Matrigel) were added to a mixed medium of DMEM and F-12 medium (DMEM/F-12 medium (ratio by volume) was 1/1) so that the concentration of FBS was 10% by volume and so that the concentration of the extracellular matrix was 2% by volume, to obtain a mixed medium.

Preparation Example 2

An anti-K5 antibody (manufactured by Abcam plc, trade name: αK5 antibody) was added to a 2% by mass BSA-containing PBSTT solution so that the concentration of the antibody was 0.33 μg/mL, to obtain a primary antibody-containing solution. K5 is a marker for an undifferentiated sebaceous gland basal cell present in the outermost layer of a sebaceous gland.

Preparation Example 3

A labelled anti-IgG antibody (manufactured by Invitrogen, trade name: α IgG antibody Alexa Fluor 647) and a nucleus-staining agent (manufactured by Invitrogen, trade name: Hoechst 33342) were added to a 2% by mass BSA-containing PBSTT solution so that the concentration of the labelled IgG antibody was 2 μg/mL and so that the concentration of the nucleus-staining agent was 10 μg/mL, to obtain a staining reagent A. The labelled anti-IgG antibody is a secondary antibody against the anti-K5 antibody used in Preparation Example 2.

Preparation Example 4

A lipid-staining reagent (manufactured by Wako Pure Chemical Corporation, trade name: Nile Red) was added to added to a 2% by mass BSA-containing PBSTT solution so that the concentration of the reagent was 10 μg/mL, to give a staining reagent B.

Preparation Example 5

A spacer (diameter: 9 mm and thickness: 0.12 mm) was stuck on a coverslip (24 mm × 55 mm), to obtain a spacer-attached coverslip.

Preparation Example 6

Under a stereoscopic microscope, a subcutaneous tissue was removed from a skin tissue by using scissors. Next, collagenous fibers were removed from the dermis in the skin tissue by using scissors and tweezers after removing the subcutaneous tissue, to obtain a sebaceous-gland-structure in which a sebaceous gland was exposed (hereinafter referred to as "sebaceous-gland-structure A"). The sebaceous-gland-structure A contains a sebaceous gland, a hair, a hair follicle and epidermis in the skin tissue, but did not substantially contain dermis and a subcutaneous tissue. In the sebaceous-gland-structure A, a sebaceous gland was exposed.

Preparation Example 7

The sebaceous-gland-structure A obtained in Preparation Example 6 was immersed in a 4% by mass PFA-containing PBS solution at 4°C for 30 minutes to fix the sebaceous-gland-structure A, thereby giving a fixed sample. The resulting fixed sample was washed by incubating the sample in a 2% by mass BSA-containing PBSTT solution at 4°C for 1 hour. Washing was carried out three times.
The fixed sample after the washing was incubated in the primary antibody-containing solution obtained in Preparation Example 2 at 4°C for 24 hours. The fixed sample after the incubation was washed by incubating the fixed sample in a 2% by mass BSA-containing PBSTT solution at 4°C for 1 hour. Washing was carried out three times.
The fixed sample after the washing was incubated in the staining reagent A obtained in Preparation Example 3 at 4°C for 12 hours, to stain the sebaceous gland basal cell and the nucleus present in the outermost layer of the sebaceous gland. The stained fixed sample was washed by incubating the sample in a 2% by mass BSA-containing PBSTT solution at 4°C for 1 hour. Washing was carried out three times.
The fixed sample after the washing was incubated in the staining reagent B obtained in Preparation Example 4 at room temperature (25°C) for 30 minutes, to stain the sebum contained in the sebaceous-gland-structure A. The stained fixed sample was washed by incubating the sample in a 2% by mass BSA-containing PBSTT solution at 4°C for 1 hour. Washing was carried out three times.
The fixed sample after the washing was subjected to incubation in a 20% by mass glycerol-containing PBS solution at 4°C for 1 hour, incubation in a 50% by mass glycerol-containing PBS solution at 4°C for 1 hour, and incubation in a 80% by mass glycerol-containing PBS solution at 4°C for 1 hour, to substitute the moisture contained in the fixed sample with glycerol.
An appropriate amount (for example, 50 μL) of an 80% by mass aqueous glycerol solution was added to a region surrounded by the spacer contained in the spacer-attached coverslip obtained in Preparation Example 5. Next, the glycerol-substituted sebaceous-gland-structure A was placed on the region surrounded by the spacer contained in the spacer-attached coverslip so that the side of the sebaceous gland was directed downwards. Thereafter, the region surrounded by the spacer contained in the spacer-attached coverslip was covered with a round-shaped coverslip (diameter: 12 mm), to obtain an observation sample.

Test Example 1

(1) Observation of Appearance of Sebaceous-gland-structure

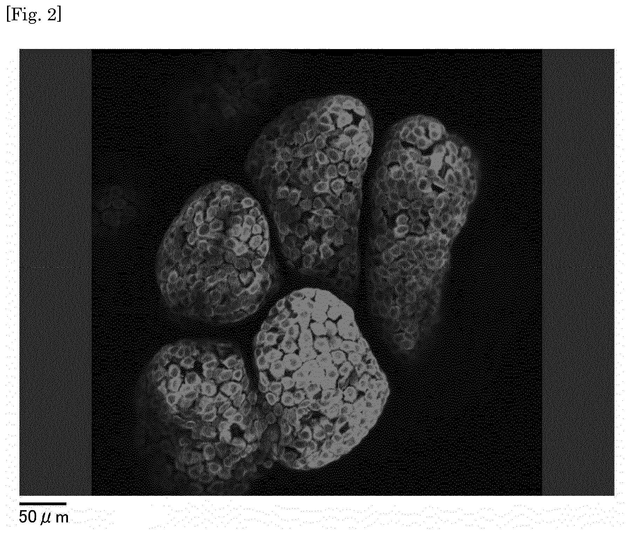

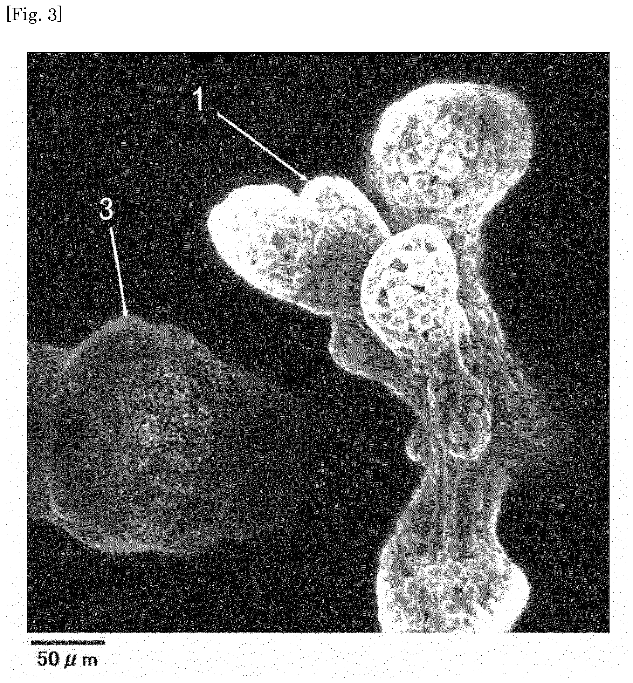

Appearance of the sebaceous-gland-structure A contained in the observation sample obtained in Preparation Example 7 was observed under a confocal microscope. In Test Example 1(1), results of observation of appearance of the sebaceous-gland-structure A contained in the observation sample obtained in Preparation Example 7 are shown in Figs. 2 and 3. The length of the scale bar shown in Fig. 2 means 50 $\mu$m. In addition, the length of the scale bar shown in Fig. 3 means 50 $\mu$m. In Fig. 3, 1 denotes a sebaceous gland, and 3 denotes a hair follicle.

From the results shown in Fig. 2, it can be seen that the sebaceous-gland-structure A has a sebaceous gland having a smooth surface covered with undifferentiated sebaceous gland basal cells. In addition, from the results shown in Fig. 3, it can be seen that a hair follicle 3 and a hair covered with a hair follicle 3 were not removed and were remained in the sebaceous-gland-structure A. Accordingly, it can be seen that the sebaceous-gland-structure A retains the structure inherent to a sebaceous gland in a living body. As described above, since the sebaceous-gland-structure A retains the structure of a sebaceous gland in a living body, it can be seen that dynamics of a sebaceous gland in a living body can be accurately observed by using the sebaceous-gland-structure A.

(2) Observation of inside of Sebaceous-gland-structure

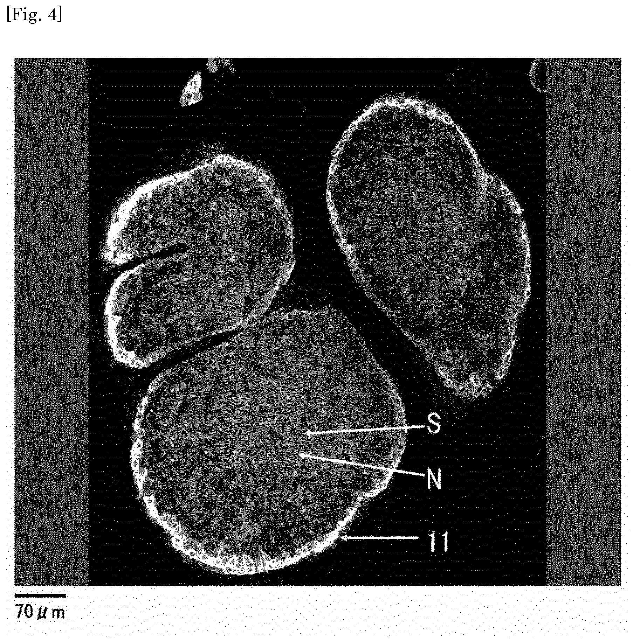

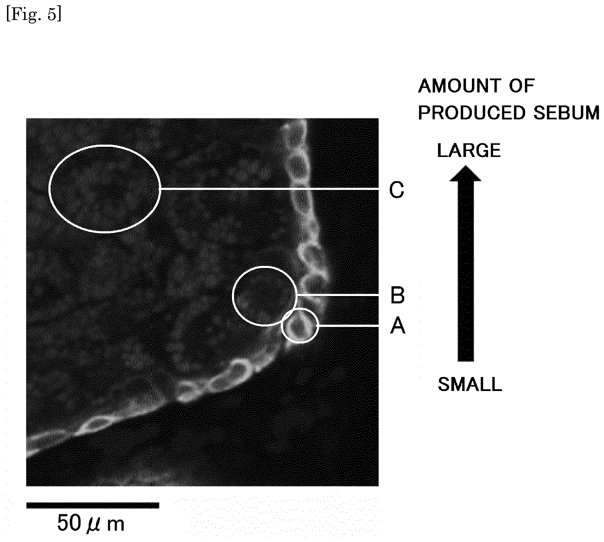

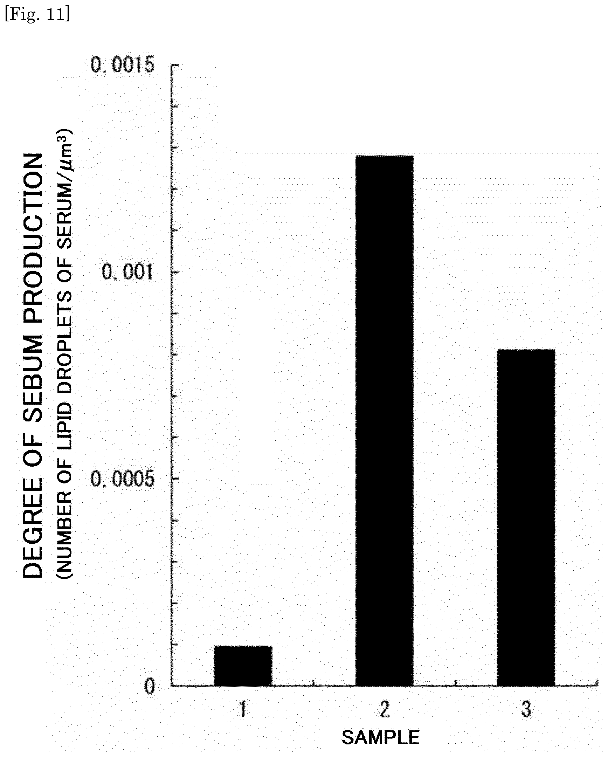

The inside of the sebaceous-gland-structure A contained in the observation sample obtained in Preparation Example 7 was observed under a confocal microscope. In Test Example 1(2), results of observation of the inside of the sebaceous-gland-structure contained in the observation sample obtained in Preparation Example 7 are shown in Fig. 4, and results of observation of a lipid droplet of sebum in the inside of the sebaceous-gland-structure contained in the observation sample obtained in Preparation Example 7 are shown in Fig. 5. The length of the scale bar shown in Fig. 4 means 70 $\mu$m, and the length of the scale bar shown in Fig. 5 means 50 $\mu$m. In Fig. 4, 11 denotes an undifferentiated sebaceous gland basal cell; and N denotes the nucleus; S denotes sebum. In addition, in Fig. 5, A denotes a lipid droplet present in the outermost layer of the sebaceous gland; B denotes the lipid droplet present in the middle layer of the sebaceous gland; and C denotes a lipid droplet present near the central part of the sebaceous gland.

From the results shown in Fig. 4, since the surface of the sebaceous gland is a smooth surface covered with undifferentiated sebaceous gland basal cells, it can be seen that the sebaceous-gland-structure A retains the structure inherent to a sebaceous gland in a living body. In addition, from the results shown in Fig. 5, it can be seen that the size of the lipid droplet in a cell becomes larger towards a portion near the center (in Fig. 5, C) from the outermost layer of the sebaceous gland (In Fig. 5, A). From these results, in the sebaceous-gland-structure A, when cultured in a suspended state in a medium, it is deduced that an undifferentiated sebaceous gland basal cell 11 is differentiated into a mature sebocyte 12 while moving from the outermost layer of a sebaceous gland 1 to the central part of the sebaceous gland 1 as shown in Fig. 1, and that a lipid droplet generated by the mature sebocyte 12 is released as sebum S from a cell debris 13 caused by death and disintegration of the mature sebocyte 12 to the outside of the skin through a hair follicle 3 which covers the hair 2. As described above, it can be seen that the sebaceous-gland-structure A retains the structure of a sebaceous gland in a living body and can reproduce the process of sebum production in a living body. Accordingly, it can be seen that, according to the sebaceous-gland-structure A, dynamics of a sebaceous gland in a living body can be accurately observed.

Example 1

(1) Preparation of Test Sample

Linoleic acid was added to the mixed medium obtained in Preparation Example 1 (hereinafter also referred to as "test sample A") so that the concentration of linoleic acid was $1 \times 10^{-4}$ M, to obtain a test sample B. In addition, linoleic acid was added to the mixed medium obtained in Preparation Example 1 so that the concentration of linoleic acid was $1 \times 10^{-3}$ M, to obtain a test sample C.

(2) Culture of Sebaceous-gland-structure and Contact of Sebaceous-gland-structure with Test Sample

One milliliter of the test sample A was added to each well of a 24-well plate. Next, the sebaceous-gland-structure A obtained in Preparation Example 6 was cultured in a suspended state in the test sample A contained in each well of the 24-well plate in the presence of 5% by volume carbon dioxide at 37°C for 24 hours, to contact the sebaceous-gland-structure A with the test sample A (Experiment number 1). In addition, the sebaceous-gland-structure A was contacted with the test sample A in the same manner as in Experiment number 1 except that in Experiment number 1, 48-hour culture was carried out in place of 24-hour culture (Experiment number 2).

The sebaceous-gland-structure A obtained in Preparation Example 6 was cultured in a suspended state in the test sample B contained in each well of the 24-well plate in the presence of 5% by volume carbon dioxide at 37°C for 24 hours, to contact the sebaceous-gland-structure A with the test sample B (Experiment number 3). In addition, the sebaceous-gland-structure A was contacted with the test sample B in the same manner as in Experiment number 3 except that in Experiment number 3, 48-hour culture was carried out in place of 24-hour culture (Experiment number 4).

The sebaceous-gland-structure A obtained in Preparation Example 6 was cultured in a suspended state in the test sample B contained in each well of the 24-well plate in the presence of 5% by volume carbon dioxide at 37°C for 24 hours, to contact the sebaceous-gland-structure A with the test sample C (Experiment number 5). In addition, the sebaceous-gland-structure A was contacted with the test sample C in the same manner as in Experiment number 5 except that in Experiment number 5, 48-hour culture was carried out in place of 24-hour culture (Experiment number 6).

(3) Preparation of Observation sample

Observation sample of each of Experiment numbers 1 to 6 was obtained in the same manner as in Preparation Example 7 except that in Preparation Example 7, the cultured sebaceous-gland-structure A obtained in Example 1(2) (each of Experiment numbers 1 to 6) was used in place of the sebaceous-gland-structure A obtained in Preparation Example 6.

(4) Observation of inside of Sebaceous-gland-structure

The inside of the sebaceous-gland-structure A contained in the observation sample of each of Experiment numbers 1 to 6 was observed under a confocal microscope in the same manner as in Test Example 1(1) except that in Test Example 1(1), the samples for observation of Experiment numbers 1 to 6 obtained in Example 1(3) were used in place of the observation sample obtained in Preparation Example 6.

(5) Observation of Process of Sebum Production

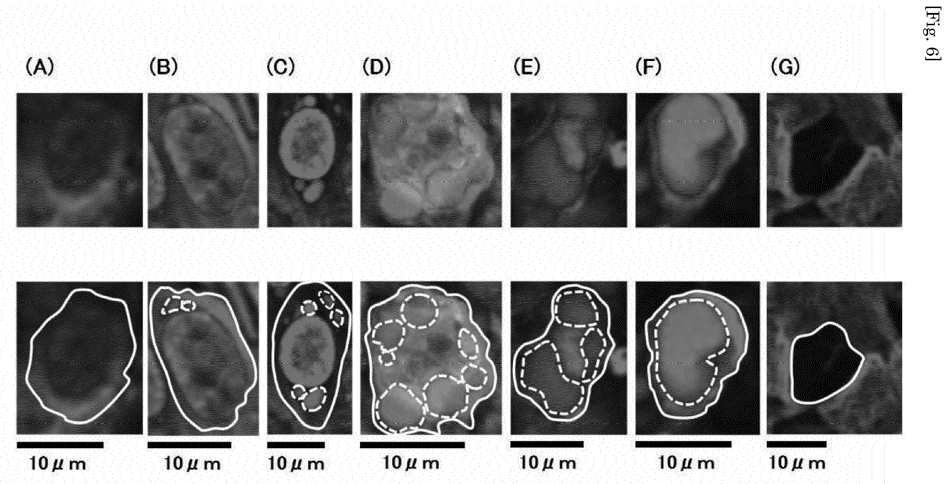

The state of the lipid droplet in the cell contained in the outermost layer of the sebaceous gland of the sebaceous-gland-structure A contained in the observation sample of each of Experiment numbers 1 to 6 was observed in the same manner as in Test Example 1(1) except that in Test Example 1(1), each of the samples for observation of Experiment numbers 1 to 6 obtained in Example 1(3) was used in place of the observation sample obtained in Preparation Example 6. Next, the state of the lipid droplet in the cell of the observed sebaceous gland was classified on the basis of (A) to (G) shown in Table 1 and thereafter observed. Classification was carried out by selecting the state of the lipid droplet apparently closest to (A) to (G) in Table 1 on the basis of the state of the lipid droplet in the observed cell. In Example 1(5), results of classification and observation of the state of the lipid droplet in the cell contained in the outermost layer of the sebaceous gland of the sebaceous-gland-structure A contained in the observation sample of each of Experiment numbers 1 to 6 are shown in Fig. 6. In the figure, the length of the scale bar shown in each of (A) to (G) means 10 μm. In addition, in the figure, portions containing cells of which differentiation progressed are shown in the order of (A) to (G). The upper side and the lower side of each of (A) to (G) show an identical portion in the sebaceous gland of the sebaceous-gland-structure A. Furthermore, in the figure, the solid line shows the outline of the cell, and the broken line shows the outline of the lipid droplet, in the lower side of (A) to (G). The state of the lipid droplet in the cell contained in the outermost layer of the sebaceous gland shown in Fig. 6(A) to (G) corresponds to the state of (A) to (G) in Table 1, respectively.

[Table 1]

| | |
|---|---|
| (A) | No lipid droplet is found in the cell. |
| (B) | A few lipid droplets are found in the cell. |
| (C) | There is a space of lipid droplets in the cell. |
| (D) | Lipid droplets in the cell start erosion of the nucleus of the cell. |
| (E) | Lipid droplets in the cell start being connected to each other. |

(continued)

| | |
|---|---|
| (F) | The cell is fully occupied by lipid droplets in its inside. |
| (G) | The cell is disappeared. |

In addition, the correspondence of the state of the lipid droplet in the cell found in the outermost layer of the sebaceous gland in the observation sample of each of Experiment numbers 1 to 6 and the state of the lipid droplet in the cell contained in the outermost layer of the sebaceous gland shown in Fig. 6 is shown in Table 2. In Table 2, "○ (open circle)" shows that a given state shown in Fig. 6 was observed, and "-" shows that the given state shown in Fig. 6 was not observed.

[Table 2]

| Experiment number | Concentration of linoleic acid (M) | Culture duration (h) | State of lipid droplet in cell contained in outermost layer of sebaceous gland | | | | | | |
|---|---|---|---|---|---|---|---|---|---|
| | | | Fig.6 (A) | Fig.6 (B) | Fig.6 (C) | Fig.6 (D) | Fig.6 (E) | Fig.6 (F) | Fig.6 (G) |
| 1 | 0 | 24 | ○ | - | - | - | - | - | - |
| 2 | 0 | 48 | ○ | - | - | - | - | - | - |
| 3 | $1 \times 10^{-4}$ | 24 | ○ | ○ | - | - | - | - | - |
| 4 | $1 \times 10^{-4}$ | 48 | ○ | ○ | ○ | - | - | - | - |
| 5 | $1 \times 10^{-3}$ | 24 | ○ | ○ | ○ | ○ | ○ | - | ○ |
| 6 | $1 \times 10^{-3}$ | 48 | ○ | ○ | ○ | ○ | ○ | ○ | ○ |

From the results shown in Fig. 6, it can be seen that the ratio of the lipid droplet occupying the components inside of the cell increases as differentiation progresses in the order of (A) to (F). In addition, in (G), it can be seen that sebum is secreted, and that cells disappear. In addition, from the results shown in Table 2, it can be seen that there is a tendency that the lower the linoleic acid concentration in the test sample is and the shorter the culture time is, the larger the number of the cells not progressing sebum production is. In addition, it can be seen that there is a tendency in sebum production in the sebaceous-gland-structure A that the higher the linoleic acid concentration in the test sample is and the longer the culture time is, the larger the number of cells progressing sebum production is, as with the sebum production in a living body. From these results, it can be seen that the sebaceous-gland-structure A can reproduce the process of sebum production in a living body. Thus, it can be seen that, according to the sebaceous-gland-structure A, dynamics of a sebaceous gland in a living body can be accurately observed.

Preparation Example 8

Linoleic acid and linoleic acid receptor antagonist (manufactured by Abcam plc, trade name: GSK-3787) as test substances were added to the mixed medium obtained in Preparation Example 1 so that the concentration of linoleic acid was $1 \times 10^{-4}$ M and so that the concentration of the test substance was 1 $\mu$M, to obtain a test sample D.

Example 2

(1) Culture of Sebaceous-gland-structure and Contact of Sebaceous-gland-structure with Test Sample

One milliliter of the test sample A was added to each well of a 24-well plate. Next, the sebaceous-gland-structure A obtained in Preparation Example 6 was cultured in a suspended state in the test sample A in each well of the 24-well plate in the presence of 5% by volume carbon dioxide at 37°C for 24 hours, to contact the sebaceous-gland-structure A with the test sample A (Experiment number 7).

In addition, the sebaceous-gland-structure A was contacted with the test sample B (Experiment number 8) or the test sample D (Experiment number 9) in the same manner as in Experiment number 7 except that in Experiment number 7, the test sample B obtained in Example 1(1) (Experiment number 8) or the test sample D obtained in Preparation Example 8 (Experiment number 9) was used in place of the test sample A.

(2) Preparation of Observation sample

Each of samples for observation of Experiment numbers 7 to 9 was obtained in the same manner as in Preparation Example 7 except that in Preparation Example 7, each of the sebum gland structure A of Experiment numbers 7 to 9 obtained in Example 2(1) was used in place of the cultured sebaceous-gland-structure A obtained in Preparation Example 6.

(3) Observation of inside of Sebaceous-gland-structure

The inside of the sebaceous-gland-structure A contained in the observation sample of each of Experiment numbers 7 to 9 was observed under a confocal microscope in the same manner as in Test Example 1(1) except that in Test Example 1(1), each of the samples for observation of Experiment numbers 7 to 9 obtained in Example 2(2) was used in place of the observation sample obtained in Preparation Example 7.

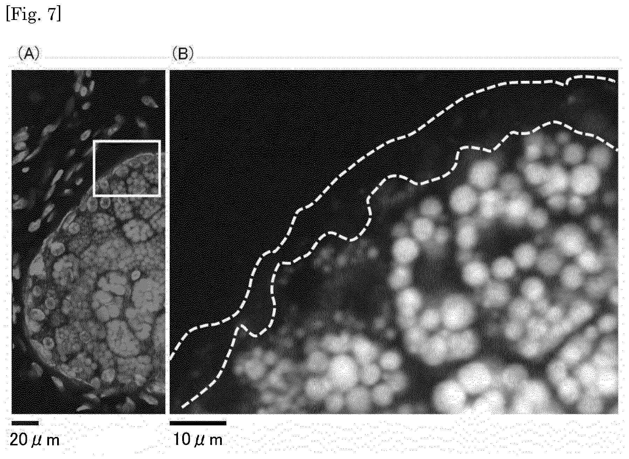

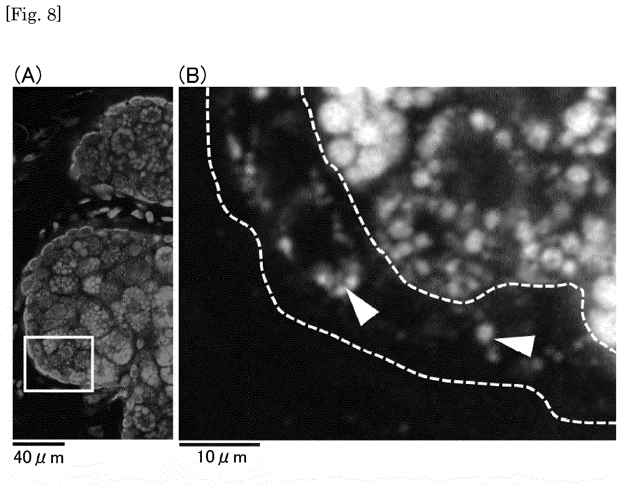

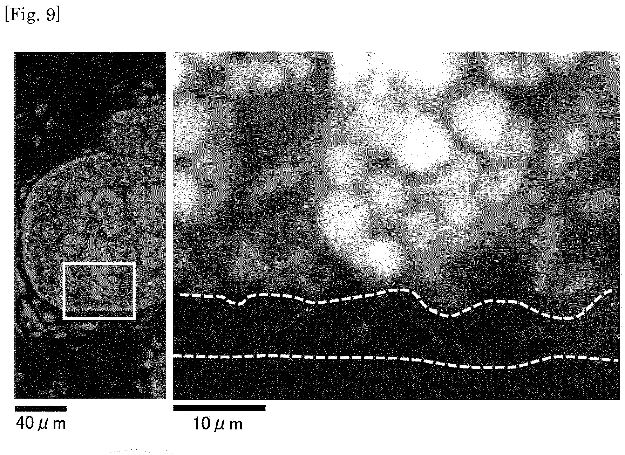

In Example 2(3), the results of observation of the inside of the sebaceous-gland-structure A contained in the observation sample of Experiment number 7 are shown in Fig. 7(A), and the enlarged part of the observed region surrounded with a frame in Fig. 7(A) are shown in Fig. 7(B). The length of the scale bar shown in Fig. 7(A) means 20 $\mu$m, and the length of the scale bar shown in Fig. 7(B) means 10 $\mu$m. In addition, in Fig. 7, the region surrounded by the broken line shows the outermost layer of the sebaceous gland. In addition, in Example 2(3), the results of observation of the inside of the sebaceous-gland-structure A contained in the observation sample of Experiment number 8 are shown in Fig. 8(A), and the enlarged part of the observed region surrounded with a frame in Fig. 8(A) are shown in Fig. 8(B). The length of the scale bar shown in Fig. 8(A) means 40 $\mu$m, and the length of the scale bar shown in Fig. 8(B) means 10 $\mu$m. In addition, in Fig. 8, the region surrounded by the broken line shows the outermost layer of the sebaceous gland, and the arrowhead shows the lipid droplet of sebum. Furthermore, in Example 2(3), the results of observation of the inside of the sebaceous-gland-structure A contained in the observation sample of Experiment number 9 are shown in Fig. 9(A), and the enlarged part of the observed region surrounded with a frame in Fig. 9(A) are shown in Fig. 9(B). The length of the scale bar shown in Fig. 9(A) means 40 $\mu$m, and the length of the scale bar shown in Fig. 9(B) means 10 $\mu$m. In addition, in Fig. 9, the region surrounded by the broken line shows the outermost layer of the sebaceous gland.

Furthermore, the state of each cell of 40 cells in the outermost layer shown in each figure was evaluated by using the results shown in Figs. 7 to 9 on the basis of the evaluation criterion described below.

<Evaluation Criterion>

0 point: No lipid droplet is found in the cell (see Fig. 6(A)).
1 point: A few lipid droplets are found in the cell (see Fig. 6 (B)).
2 points: There is a space of lipid droplets in the cell (see fig. 6(C)).
3 points: Lipid droplets in the cell start erosion of the nucleus of the cell (see Fig. 6(D)).
4 points: Lipid droplets in the cell start being connected to each other (see Fig. 6(E)).
5 points: The cell is fully occupied by lipid droplets in its inside (see Fig. 6(F)).
6 points: The cell is disappeared (see Fig. 6(G)).

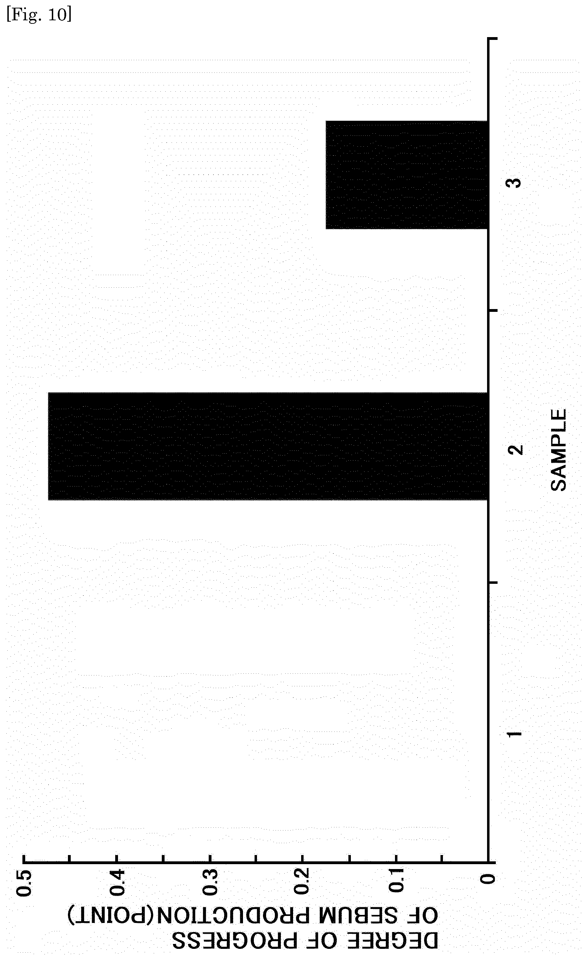

Next, the degree of progress of sebum production in the sebaceous gland cell contained in the sebaceous-gland-structure A was evaluated by calculating the mean value of the points in the 40 cells in the outermost layer in Figs. 7 to 9. In Example 2(3), the results of examining the relationship between the kind of the sample and the degree of progress of sebum production are shown in Fig. 10. In the figure, lane 1 shows the degree of progress of sebum production in the sebaceous-gland-structure A contacted with the test sample A containing no linoleic acid (Experiment number 7); lane 2 shows the degree of progress of sebum production in the sebaceous-gland-structure A contacted with the test sample B containing linoleic acid (Experiment number 8); and lane 3 shows the degree of progress of sebum production in the sebaceous-gland-structure A contacted with the test sample D containing linoleic acid and linoleic acid receptor antagonist (Experiment number 9).

From the results shown in Figs. 7 and 10, it can be seen that no lipid droplet of sebum was found in the outermost layer of the sebaceous-gland-structure A contacted with the test sample A containing no linoleic acid (Experiment number 7). However, from the results shown in Figs. 8 and 10, it can be seen that lipid droplets of sebum were found in the outermost layer of the sebaceous-gland-structure A contacted with the test sample B containing linoleic acid (Experiment number 8). On the other hand, from the results shown in Figs. 9 and 10, it can be seen that no lipid droplet of sebum was found in the outermost layer of the sebaceous-gland-structure A contacted with the test sample D containing linoleic acid and linoleic acid receptor antagonist (Experiment number 9).

Preparation Example 9

A subcutaneous tissue was removed from a skin tissue by using scissors under a stereoscopic microscope. Next, the skin tissue from which the subcutaneous tissue was removed was incubated in a suspended state in a dispase-containing PBS solution contained in a 60 mm-diameter dish at 4°C for 16 hours. The dermis was removed from the incubated skin tissue, to obtain a structure containing epidermis, a hair, a hair follicle and a sebaceous gland.

After damaging the periphery of the root of the hair follicle having the sebaceous gland by the tip of tweezers, the hair follicle having the sebaceous gland was pulled from the structure, thereby giving a sebaceous-gland-structure containing a sebaceous gland and a hair follicle (hereinafter referred to as "sebaceous-gland-structure B"). The sebaceous-gland-structure B contained a sebaceous gland, a hair, a hair follicle and epidermis in a skin tissue, but did not substantially contain the dermis and the subcutaneous tissue.

Example 3

(1) Culture of Sebaceous-gland-structure

The sebaceous-gland-structure is contacted with a test sample in the same manner as in Example 2(1) except that in Example 2(1), the sebaceous-gland-structure B obtained in Preparation Example 9 is used in place of the sebaceous-gland-structure A obtained in Preparation Example 6.

(2) Preparation of Observation sample

An observation sample is obtained in the same manner as in Preparation Example 7 except that in Preparation Example 7, the cultured sebaceous-gland-structure B obtained in Example 3(1) is used in place of the sebaceous-gland-structure A obtained in Preparation Example 6.

(3) Observation of Sebaceous-gland-structure

The sebaceous-gland-structure B is observed in the same manner as in Test Example 1(1) except that in Test Example 1(1), the observation sample obtained in Example 3(2) is used in place of the observation sample obtained in Preparation Example 7. As a result, it can be seen that dynamics of the sebaceous gland can be accurately observed, when the sebaceous gland of the sebaceous-gland-structure B is small. On the other hand, it can be seen that it is difficult to accurately observe dynamics of the sebaceous gland as compared with the case where the sebaceous-gland-structure A obtained in Preparation Example 6 was used, when the sebaceous gland of the sebaceous-gland-structure B is large.

Example 4

(1) Preparation of Test Sample

Linoleic acid and phytic acid as test substances were added to the mixed medium obtained in Preparation Example 1 (test sample A) so that the concentration of linoleic acid was $1 \times 10^{-4}$ M and so that the concentration of the test sample was 100 $\mu$M, to obtain a test sample E.

(2) Culture of Sebaceous-gland-structure and Contact of Sebaceous-gland-structure with Test Sample

One milliliter of the test sample A was added to each well of a 24-well plate. Next, the sebaceous-gland-structure A obtained in Preparation Example 6 was cultured in a suspended state in the test sample A contained in each well of the 24-well plate in the presence of 5% by volume carbon dioxide at 37°C for 24 hours, to contact the sebaceous-gland-structure A with the test sample A (Experiment number 10).

In addition, the sebaceous-gland-structure A was contacted with the test sample B (Experiment number 11) or the test sample E (Experiment number 12) in the same manner as in Experiment number 10 except that in Experiment number 10, the test sample B obtained in Example 1(1) (Experiment number 11) or the test sample E obtained in Example 4(1) (Experiment number 12) was used in place of the test sample A.

(3) Preparation of Observation sample

Each of observation samples of Experiment numbers 10 to 12 was obtained in the same manner as in Preparation Example 7 except that in Preparation Example 7, the cultured sebaceous-gland-structure A obtained in Example 4(2) (Experiment numbers 10 to 12) was used in place of the cultured sebaceous-gland-structure A obtained in Preparation Example 6.

(4) Observation of Sebaceous-gland-structure

The appearance of the sebaceous-gland-structure A contained in the observation sample of each of Experiment numbers 10 to 12 obtained in Example 4(3) was observed under a confocal microscope by using image analysis software (manufactured by Bitplane AG, trade name: Imaris), thereby giving three-dimensional image of the sebaceous-gland-structure A. Next, information of the outermost layer of the sebaceous-gland-structure A was extracted from the data of the resulting three-dimensional image by using image analysis software (manufactured by Bitplane AG, trade name: Imaris). Thereafter, the whole volume of the cells present in the outermost layer was calculated from the extracted information, and thereafter the sebum droplets contained in the outermost layer were counted. Using the whole volume of the cells present in the outermost layer and the number of lipid droplets of the sebum, the degree of sebum production was calculated according to Formula (I):

$$[\text{Degree of Sebum production}]$$

$$= [\text{Number of lipid droplets of sebum}] \, / \, [\text{Whole volume of cells present in outermost layer}] \qquad (I)$$

The results of examining the relationship of the kind of the sample and the degree of sebum production are shown in Fig. 11. In the figure, lane 1 shows the degree of sebum production in the sebaceous-gland-structure A contacted with the test sample not containing linoleic acid; lane 2 shows the degree of sebum production in the sebaceous-gland-structure A contacted with the test sample B containing linoleic acid; and lane 3 shows the degree of progress of sebum production in the sebaceous-gland-structure A contacted with the test sample E containing linoleic acid and phytic acid.

From the results shown in Fig. 11, it can be seen that the degree of sebum production in the sebaceous-gland-structure A contacted with the test sample B containing linoleic acid was higher as compared with the degree of sebum production in the sebaceous-gland-structure A contacted with the test sample A not containing linoleic acid. Accordingly, it can be seen that linoleic acid has a sebum production-promoting action. On the other hand, it can be seen that the degree of sebum production in the sebaceous-gland-structure A contacted with the test sample E containing linoleic acid and phytic acid was lower as compared with the degree of sebum production in the sebaceous-gland-structure A contacted with the test sample containing linoleic acid. Accordingly, it can be seen that phytic acid has a sebum production-inhibiting action. From these results, it can be seen that a sebum production-regulating action of a test substance can be accurately evaluated by the sebaceous-gland-structure A.

As explained above, it can be seen that, by using a sebaceous-gland-structure derived from a skin tissue, in which all or a portion of each of dermis and a subcutaneous tissue has been removed from the skin tissue, dynamics of a sebaceous gland can be accurately observed, and whether or not the test substance is a substance having a sebum production-regulating action can be accurately evaluated. Accordingly, it is expected that the method for observing a sebaceous gland and the method for evaluating a test substance of the present invention are used for development of external preparations suitable for a human in need of regulation of sebum production such as a cosmetic.

DESCRIPTION OF SYMBOLS

1: sebaceous gland
2: hair
3: hair follicle
4: epidermis
11: undifferentiated sebaceous gland basal cell
12: mature sebocyte
13: cell debris
S: sebum
N: nucleus

**Claims**

1.  A method for observing a sebaceous gland, comprising the steps of:

    (I) culturing, in a suspended state in a medium, a sebaceous-gland-structure derived from a skin tissue, in which all or a portion of each of dermis and a subcutaneous tissue has been removed from the skin tissue, and
    (II) observing the resulting cultured sebaceous-gland-structure in step (I).

2.  The method for observing a sebaceous gland according to claim 1, further comprising a step of removing all or the portion of each of the dermis and the subcutaneous tissue from an isolated skin tissue, to obtain the sebaceous-gland-structure, before carrying out the step (I).

3.  A method for evaluating a sebum production-regulating action of a test substance, comprising the steps of:

    (A) culturing, in a suspended state in a medium in the absence of the test substance, a sebaceous-gland-structure derived from a skin tissue, in which all or a portion of each of dermis and a subcutaneous tissue has been removed from the skin tissue,
    (B) culturing, in a suspended state in a medium in the presence of the test substance, a sebaceous-gland-structure derived from a skin tissue, in which all or a portion of each of dermis and a subcutaneous tissue has been removed from the skin tissue, and
    (C) observing the resulting cultured sebaceous-gland-structure (A) in the step (A) and the resulting cultured sebaceous-gland-structure (B) in the step (B), and evaluating the sebum production-regulating action of the test substance on the basis of difference in dynamics of the sebaceous gland in the sebaceous-gland-structure (A) and dynamics of the sebaceous gland in the sebaceous-gland-structure (B).

[Fig. 1]

[Fig. 2]

50 $\mu$ m

[Fig. 3]

50 μm

[Fig. 4]

70 μm

[Fig. 5]

AMOUNT OF
PRODUCED SEBUM

LARGE

C

B
A

SMALL

50 μ m

[Fig. 6]

[Fig. 7]

(A)　　　　　(B)

20 μm　　10 μm

[Fig. 8]

(A)

(B)

40 μm

10 μm

[Fig. 9]

40 μ m

10 μ m

[Fig. 10]

[Fig. 11]

## INTERNATIONAL SEARCH REPORT

| International application No. |
|---|
| PCT/JP2018/007559 |

| A. CLASSIFICATION OF SUBJECT MATTER |
|---|
| Int. Cl. G01N33/50(2006.01)i, C12Q1/02(2006.01)i, G01N33/15(2006.01)i |

According to International Patent Classification (IPC) or to both national classification and IPC

| B. FIELDS SEARCHED |
|---|
| Minimum documentation searched (classification system followed by classification symbols) |
| Int. Cl. G01N33/50, C12Q1/02, G01N33/15 |

Documentation searched other than minimum documentation to the extent that such documents are included in the fields searched
Published examined utility model applications of Japan     1922-1996
Published unexamined utility model applications of Japan   1971-2018
Registered utility model specifications of Japan           1996-2018
Published registered utility model applications of Japan   1994-2018

Electronic data base consulted during the international search (name of data base and, where practicable, search terms used)
JSTPlus/JMEDPlus/JST7580 (JDreamIII)

| C. DOCUMENTS CONSIDERED TO BE RELEVANT |
|---|

| Category* | Citation of document, with indication, where appropriate, of the relevant passages | Relevant to claim No. |
|---|---|---|
| X, Y | JP 05-268949 A (NIPPON ZEON CO., LTD.) 19 October 1993, entire text, in particular, claims, paragraphs [0006]-[0014], [0021]-[0032] (Family: none) | 1-2/3 |
| Y | JP 2011-523548 A (GALDERMA RESEARCH & DEVELOPMENT) 18 August 2011, entire text, in particular, claims, paragraphs [0009]-[0014], [0091]-[0099] & US 2011/0150773 A1, entire text, in particular, claims, paragraphs [0007]-[0012], [0102]-[0110] & WO 2009/135911 A1 & EP 2286242 A1 | 3 |

☒ Further documents are listed in the continuation of Box C.　　☐ See patent family annex.

| * Special categories of cited documents: | |
|---|---|
| "A" document defining the general state of the art which is not considered to be of particular relevance | "T" later document published after the international filing date or priority date and not in conflict with the application but cited to understand the principle or theory underlying the invention |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 22.05.2018 | 05.06.2018 |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

| International application No. |
|---|
| PCT/JP2018/007559 |

C (Continuation).    DOCUMENTS CONSIDERED TO BE RELEVANT

| Category* | Citation of document, with indication, where appropriate, of the relevant passages | Relevant to claim No. |
|---|---|---|
| Y | JP 2011-523354 A (GALDERMA RESEARCH & DEVELOPMENT) 11 August 2011, entire text, in particular, claims, paragraphs [0030], [0081]-[0090] & US 2011/0165168 A1, entire text, in particular, claims, paragraphs [0029]-[0033], [0094]-[0102] & WO 2009/135906 A1 & EP 2276854 A1 | 3 |
| A | JP 2015-530122 A (CHILDREN'S HOSPITAL MEDICAL CENTER) 15 October 2015, entire text & US 2015/0268254 A1 & WO 2014/055815 A1 & EP 2904095 A1 | 1-3 |
| A | JP 2013-032331 A (KOSE CORP.) 14 February 2013, entire text (Family: none) | 1-3 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013032331 A